# EUROPÄISCHE PATENTANMELDUNG

(11) **EP 0 924 246 A1**
(43) Veröffentlichungstag der Anmeldung: **23.06.1999**
(21) Anmeldenummer: 98120901.8
(22) Anmeldetag: 04.11.1998
(51) Int. Cl.: C08K 5/3417, C09K 15/30, C09D 7/12, A61K 31/40

(54) **Verwendungen von Isoindolinonderivaten**

(30) Priorität: 19.12.1997 DE 19756778
(71) Anmelder: BASF AKTIENGESELLSCHAFT, 67056 Ludwigshafen (DE)
(72) Erfinder: Habeck, Thorsten Dr., 67149 Meckenheim (DE); Haremza, Sylke Dr., 69151 Neckargemünd (DE); Trauth, Hubert, 67373 Dudenhofen (DE); Schehlmann, Volker Dr., 67354 Römerberg (DE); Westenfelder, Horst, 67435 Neustadt (DE)

(57) **Zusammenfassung**

Verwendung von Isoindolinonderivaten der allgemeinen Formel I, 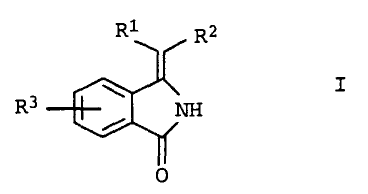 in der die Variablen folgende Bedeutung haben:
- R¹: Wasserstoff, COOR⁴, COR⁴, CONR⁴R⁵, CN, C₁-C₂₀-Alkyl, C₂-C₁₀-Alkenyl, C₃-C₁₀-Cycloalkyl, C₃-C₁₀-Cycloalkenyl, Aryl, Heteroaryl, gegebenenfalls substituiert;
- R¹ und R²: gemeinsam C₇-C₁₀-Bicycloalkyl, C₇-C₁₀-Bicycloalkenyl;
- R³: Wasserstoff, C₁-C₂₀-Alkyl, C₂-C₁₀-Alkenyl, C₃-C₁₀-Cycloalkyl, C₃-C₁₀-Cycloalkenyl, C₁-C₁₂-Alkoxy, C₁-C₂₀-Alkoxycarbonyl, C₁-C₁₂-Alkylamino, C₁-C₁₂-Dialkylamino, Aryl, Heteroaryl, gegebenenfalls substituiert, wasserlöslich machende Substituenten, ausgewählt aus der Gruppe bestehend aus Carboxylat-, Sulfonat- oder Ammoniumresten;
- R⁴ und R⁵: unabhängig voneinander Wasserstoff, C₁-C₂₀-Alkyl, C₂-C₁₀-Alkenyl, C₃-C₁₀-Cycloalkyl, C₃-C₁₀-Cycloalkenyl; Aryl, Heteroaryl, gegebenenfalls substituiert;
als Stabilisatoren für organische Materialien.

## Beschreibung

Die vorliegende Erfindung betrifft die Verwendung von Isoindolinonderivaten als Stabilisatoren für organische Materialien.

Organisches Material, insbesondere Kunststoffe und Lacke, wird bekanntermaßen sehr schnell, vor allem durch Einwirkung von Licht, zerstört. Diese Zerstörung zeigt sich üblicherweise in Vergilbung, Verfärbung, Rißbildung oder Versprödung des Materials. Mit den bisher verwendeten Stabilisatoren und Lichtschutzmitteln konnte kein zufriedenstellender Schutz gegen die Zerstörung von organischem Material durch Licht, Sauerstoff und Wärme erzielt werden.

So werden beispielsweise in der EP-A-057 160 o-Hydroxyphenylbenztriazol-Derivate der allgemeinen Formel 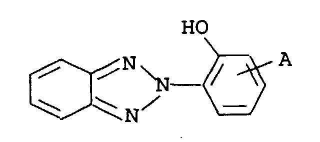 in der A z.B. für Polyethylenoxycarbonylalkyl-Reste oder zwei o-Hydroxyhenylbenztriazol-Systeme verbindende Polyethylenoxycarbonylalkylen-Brückenglieder steht, als UV-Absorber für Kunststoffe und Lacke empfohlen. Die genannten o-Hydroxphenylbenztriazol-Derivate weisen zwar die gewünschten spektroskopischen Eigenschaften auf (starke Absorptionsbanden im Bereich von 280 bis 360 nm), genügen jedoch hinsichtlich ihrer Stabilisierungs- bzw. Lichtschutzwirkung nicht den heute gestellten Anforderungen.

Aufgabe der vorliegenden Erfindung war es daher, Stabilisatoren bzw. Lichtschutzmittel bereitzustellen, die einen wirkungsvollen Schutz für organisches Material mit sich bringen.

Diese Aufgabe wurde erfindungsgemäß gelöst durch die Verwendung von Isoindolinonderivaten der allgemeinen Formel I, 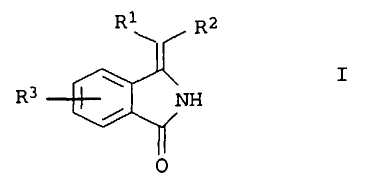 in der die Variablen unabhängig voneinander folgende Bedeutung haben:
- R¹: Wasserstoff, COOR⁴, COR⁴, CONR⁴R⁵, CN, C₁-C₂₀-Alkyl, C₂-C₁₀-Alkenyl, C₃-C₁₀-Cycloalkyl, C₃-C₁₀-Cycloalkenyl, Aryl, Heteroaryl, gegebenenfalls substituiert;
- R²: COOR⁴, COR⁴, CONR⁴R⁵, CN;
- R¹ und R²: gemeinsam C₇-C₁₀-Bicycloalkyl, C₇-C₁₀-Bicycloalkenyl;
- R³: Wasserstoff, C₁-C₂₀-Alkyl, C₂-C₁₀-Alkenyl, C₃-C₁₀-Cycloalkyl, C₃-C₁₀-Cycloalkenyl, C₁-C₁₂-Alkoxy, C₁-C₂₀-Alkoxycarbonyl, C₁-C₁₂-Alkylamino, C₁-C₁₂-Dialkylamino, Aryl, Heteroaryl, gegebenenfalls substituiert, wasserlöslich machende Substituenten, ausgewählt aus der Gruppe bestehend aus Carboxylat-, Sulfonat- oder Alkylammoniumresten;
- R⁴ und R⁵: Wasserstoff, C₁-C₂₀-Alkyl, C₂-C₁₀-Alkenyl, C₃-C₁₀-Cycloalkyl, C₃-C₁₀-Cycloalkenyl; Aryl, Heteroaryl, gegebenenfalls substituiert;
als Stabilisatoren für organische Materialien.

Als Alkylreste für R¹ und R³ bis R⁵ seien verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Alkenylreste für R¹ und R³ bis R⁵ seien verzweigte oder unverzweigte C₂-C₁₀-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Cycloalkylreste für R¹ und R³ bis R⁵ seien bevorzugt verzweigte oder unverzweigte C₃-C₁₀-Cycloalkylreste wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl genannt.

Als Cycloalkenylreste für R¹ und R³ bis R⁵ seien bevorzugt verzweigte oder unverzweigte, C₃-C₁₀-Cycloalkenylreste mit einer oder mehreren Doppelbindungen wie Cyclopropenyl, Cyclobutenyl, Cyclopentenyl, Cyclopentadienyl, Cyclohexenyl, 1,3-Cyclohexadienyl, 1,4-Cyclohexadienyl, Cycloheptenyl, Cycloheptatrienyl, Cyclooctenyl, 1,5-Cyclooctadienyl, Cyclooctatetraenyl, Cyclononenyl oder Cyclodecenyl genannt.

Die Cycloalkenyl- und Cycloalkylreste können ggf. mit einem oder mehreren, z.B. ein bis drei Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Bicycloalkyl- oder Bicycloalkenylreste, die gemeinsam von R¹ und R² gebildet werden können, seien gesättigte oder ungesättigte C₇-C₁₀ bicyclische Ringsysteme, insbesondere bicyclische Terpene wie Pinan-, Pinen-, Bornan-, Campherderivate oder auch Adamantan genannt.

Als Alkoxyreste für R³ kommen solche mit 1 bis 12 C-Atomen, vorzugsweise mit 1 bis 8 C-Atomen in Betracht.

Beispielsweise sind zu nennen:
- Methoxy-: Ethoxy-
- iso-Propoxy-: n-Propoxy-
- 1-Methylpropoxy-: n-Butoxy-
- n-Pentoxy-: 2-Methylpropoxy-
- 3-Methylbutoxy-: 1,1-Dimethylpropoxy-
- 2,2-Dimethylpropoxy-: Hexoxy-
- 1-Methyl-1-ethylpropoxy-: Heptoxy-
- Octoxy-: 2-Ethylhexoxy-

Alkoxycarbonylreste für R³ sind z.B Ester, die die oben genannten Alkoxyreste oder Reste von höheren Alkoholen z.B. mit bis zu 20 C-Atomen, wie iso-C₁₅-Alkohol, enthalten.

Als Mono- oder Dialkylaminoreste für R³ kommen solche in Betracht, die Alkylreste mit 1 bis 12 C-Atomen enthalten, wie z.B. Methyl-, n-Propyl-, n-Butyl-, 2-Methylpropyl-, 1,1-Dimethylpropyl-, Hexyl-, Heptyl-, 2-Ethylhexyl-, Isopropyl-, 1-Methylpropyl-, n-Pentyl-, 3-Methylbutyl-, 2,2-Dimethylpropyl-, 1-Methyl-1-ethylpropyl- und Octyl.

Unter Aryl sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind ggf. substituiertes Phenyl, Methoxyphenyl und Naphthyl.

Heteroaryl-Reste sind vorteilhafterweise einfache oder kondensierte aromatische Ringsysteme mit einem oder mehreren heteroaromatischen 3- bis 7-gliedrigen Ringen. Als Heteroatome können ein oder mehrere Stickstoff-, Schwefel- und/oder Sauerstoffatome im Ring oder Ringsystem enthalten sein.

Hydrophile d.h. die Wasserlöslichkeit der Verbindungen der Formel I ermöglichende Reste für R³ sind z.B. Carboxy- und Sulfoxyreste und insbesondere deren Salze mit beliebigen physiologisch verträglichen Kationen, wie die Alkalisalze oder wie die Trialkylammoniumsalze, wie Tri-(hydroxyalkyl)-ammoniumsalze oder die 2-Methylpropan-1-ol-2-ammoniumsalze. Ferner kommen Alkylammoniumreste mit beliebigen physiologisch verträglichen Anionen in Betracht.

Bevorzugt sind solche Verbindungen der Formel I, in der die Variablen unabhängig voneinander folgende Bedeutung haben:
- R¹ und R²: COOR⁴, COR⁴, CONR⁴R⁵, CN;
- R³: Wasserstoff, C₁-C₈-Alkyl, C₁-C₈-Alkoxy, C₁-C₂₀-Alkoxycarbonyl, C₁-C₁₂-Alkylamino, C₁-C₁₂-Dialkylamino, Aryl, Heteroaryl, gegebenenfalls substituiert, wasserlöslich machende Substituenten, ausgewählt aus der Gruppe bestehend aus Carboxylat-, Sulfonat- oder Alkylammoniumresten;
- R⁴ und R⁵: Wasserstoff, C₁-C₈-Alkyl, C₅-C₆-Cycloalkyl, Phenyl.

Besonders bevorzugt sind solche Verbindungen der Formel I, in der die Variablen unabhängig voneinander folgende Bedeutung haben:
- R¹ und R²: COOR⁴, COR⁴, CONR⁴R⁵, CN;
- R³: Wasserstoff, C₁-C₈-Alkyl, C₁-C₈-Alkoxy, C₁-C₂₀-Alkoxycarbonyl, C₁-C₁₂-Alkylamino, C₁-C₁₂-Dialkylamino, Aryl, Heteroaryl, gegebenenfalls substituiert, Sulfonatrest;
- R⁴ und R⁵: Wasserstoff, C₁-C₈-Alkyl.

Als Alkylreste für R³ bis R⁵ seien besonders bevorzugt verzweigte oder unverzweigte C₁-C₈-Alkylketten, insbesondere Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethyl-hexyl oder n-Octyl genannt.

Als Cycloalkylreste für R⁴ und R⁵ seien besonders bevorzugt verzweigte oder unverzweigte C₅-C₆-Cycloalkylketten wie Cyclopentyl und Cyclohexyl genannt.

Als Alkoxyreste für R³ kommen besonders bevorzugt solche mit 1 bis 8 C-Atomen, vorzugsweise mit 1 bis 6 C-Atomen in Betracht.

Beispielsweise sind zu nennen:
- Methoxy-: Ethoxy-
- iso-Propoxy-: n-Propoxy-
- 1-Methylpropoxy-: n-Butoxy-
- n-Pentoxy-: 2-Methylpropoxy-
- 3-Methylbutoxy-: 1,1-Dimethylpropoxy-
- 2,2-Dimethylpropoxy-: Hexoxy-

Ganz besonders bevorzugt sind solche Verbindungen der Formel Ia, 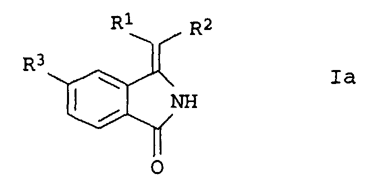 in der die Variablen unabhägig voneinander folgende Bedeutung haben:
- R¹ und R²: COOR⁴, COR⁴, CONR⁴R⁵, CN;
- R³: Wasserstoff, C₁-C₈-Alkyl, C₁-C₄-Alkoxy, C₁-C₈-Alkoxycarbonyl, Sulfonatrest;
- R⁴ und R⁵: Wasserstoff, C₁-C₈-Alkyl.

Als Alkoxyreste für R³ kommen besonders bevorzugt solche mit 1 bis 4 C-Atomen in Betracht.

Beispielsweise sind zu nennen:
- Methoxy-: Ethoxy-
- iso-Propoxy-: n-Propoxy-
- 1-Methylpropoxy-: n-Butoxy-

Alkoxycarbonylreste für R³ sind Ester, die C₁-C₈-Alkoxyrest enthalten.

Insbesondere sind folgende Alkoxyreste zu nennen:
- Methoxy-: Ethoxy-
- iso-Propoxy-: n-Propoxy-
- 1-Methylpropoxy-: n-Butoxy-
- n-Pentoxy-: 2-Methylpropoxy-
- 3-Methylbutoxy-: 1,1-Dimethylpropoxy-
- 2,2-Dimethylpropoxy-: Hexoxy-
- 1-Methyl-1-ethylpropoxy-: Heptoxy-
- Octoxy-: 2-Ethylhexoxy-

Bezüglich der räumlichen Stellung der Substituenten R¹ und R² an der C-C-Doppelbindung umfaßt die Struktur I sowohl die jeweiligen E- als auch die Z-Isomeren. Selbstverständlich können auch Mischungen beider Isomeren auftreten.

Die erfindungsgemäßen Isoindolinonderivate I eignen sich in hervorragender Weise zum Stabilisieren von organischem Material u.a. gegen die Einwirkung von Licht, Sauerstoff und Wärme. Sie werden dem zu stabilisierenden organischen Material in einer Konzentration von 0,01 bis 10 Gew.-%, bevorzugt 0,01 bis 5 Gew.-%, besonders bevorzugt von 0,02 bis 2 Gew.-%, bezogen auf das organische Material, vor, während oder nach seiner Herstellung zugesetzt.

Unter organischem Material sind beispielsweise photographische Aufzeichnungsmaterialien, insbesondere photographische Emulsionen oder Vorprodukte für Kunststoffe und Lacke, insbesondere jedoch Kunststoffe und Lacke selbst zu verstehen.

Unter organischem Material sind aber auch kosmetische Zubereitungenn wie beispielsweise Salben und Lotionen sowie Arzneimittelzubereitungen wie z.B. Pillen und Zäpfchen zu verstehen.

Gegenstand der vorliegenden Erfindung ist außerdem gegen die Einwirkung von Licht, Sauerstoff und Wärme stabilisiertes organisches Material, insbesondere Kunststoffe und Lacke, enthaltend 0,01 bis 10 Gew.-%, bevorzugt 0,01 bis 5 Gew.-%, besonders bevorzugt von 0,02 bis 2 Gew.-%, bezogen auf die Menge des organischen Materials, eines oder mehrerer Isoindolinonderivate der Formel I.

Zur Vermischung der erfindungsgemäßen Verbindungen I vor allem mit Kunststoffen können alle bekannten Vorrichtungen und Methoden zum Einmischen von Stabilisierungsmitteln oder anderen Zusätzen in Polymere angewandt werden.

Das durch die erfindungsgemäßen Verbindungen I stabilisierte organische Material kann gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Verbindungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionylethyl]isocyanurat, 1,3,5-Tris-(2,6-dimethyl-3-hydroxy-4-tert.-butylbenzyl)-isocyanurat und Pentaerythrit-tetrakis-[β-(3,5-di-tert.-butyl-4-hydroxy-phenyl)-propionat] erwähnt.

Als phosphorhaltige Antioxidantien kommen beispielsweise Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) genannt.

Weitere Antioxidatien und Lichtstabilisatoren, die zusammen mit den Verbindungen I verwendet werden können, sind z.B. 2-(2'-Hydroxyphenyl)-benztriazole, 2-Hydroxybenzophenone, Arylester von Hydroxybenzoesäuren, α-Cyanozimtsäurederivate, Benzimidazolcarbonsäureanilide, Nickelverbindungen oder Oxalsäuredianilide.

Eine besonders gute Stabilisierung erhält man, wenn zu den Verbindungen I noch mindestens ein Lichtstabilisator aus der Verbindungsklasse der sterisch gehinderten Amine in üblicher Konzentration zugesetzt wird.

Als sterisch gehinderte Amine kommen z.B. in Betracht: Bis-(2,2,6,6-tetramethylpiperidyl)-sebacat, Bis-(1,2,2,6,6-pentamethylpiperidyl)-sebacat, das Kondensationsprodukt von 1-Hydroxyethyl-2,2,6,6-tetramethyl-4-hydroxypiperidin und Bernsteinsäure, das Kondensationsprodukt von N,N'-Di-(2,2,6,6-Tetramethylpiperidyl)-hexamethylendiamin und 4-tert.-Octylamino-2,6-dichlor-1,3,5-triazin, Tris-(2,2,6,6-Tetramethylpiperidyl)-nitrilotriacetat, Tetrakis-(2,2,6,6-tetramethyl-4-piperidyl)-1,2,3,4-butan-tetracarboxylat, 1,1'-(1,2-Ethandiyl)-bis-(3,3,5,5-tetramethylpiperazinon), die Kondensationsprodukte von 4-Amino-2,2,6,6-tetramethylpiperidinen und Tetramethylolacetylendiharnstoffen.

Als Kunststoffe, die durch die erfindungsgemäßen Verbindungen I stabilisiert werden können, seien beispielsweise genannt:
Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol und Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);
halogenhaltige Polymere, wie z.B. Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acylderivaten oder Acetalen ableiten, z.B. Polyvinylalkohol und Polyvinylacetat;
Polyurethane, Polyamide, Polyharnstoffe, Polyester, Polycarbonate, Polysulfonate, Polyethersulfone und Polyetherketone.

Weiterhin können mit den erfindungsgemäßen Verbindungen I Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die Verbindungen I können in fester oder gelöster Form dem Lack zugesetzt werden. Ihre gute Löslichkeit in Lacksystemen ist dabei von besonderem Vorteil.

Auch bei der Verwendung als Stabilisatoren in Lacken können die bereits aufgeführten zusätzlichen Additive, insbesondere Antioxidantien und Lichtstabilisatoren, mitverwendet werden.

Die erfindungsgemäßen Verbindungen I zeichnen sich durch eine gute Verträglichkeit mit den üblichen Kunststoffarten und durch eine gute Löslichkeit in den üblichen Lacksystemen und in den üblichen kosmetischen Ölen aus. Sie haben in der Regel keine oder nur eine sehr geringe Eigenfarbe, sind bei den üblichen Kunststoff- und Lack-Verarbeitungstemperaturen stabil und nicht flüchtig, zeigen eine nur geringe Migrationsneigung und bewirken vor allen Dingen eine lange Schutzdauer der mit ihnen behandelten organischen Materialien.

Weiterhin eignen sich die erfindungsgemäßen Isoindolinonderivate I auch als photostabile UV-Filter in kosmetischen und pharmazeutischen Zubereitungen zum Schutz der menschlichen Haut oder menschlicher Haare gegen Sonnenstrahlen aber auch gegen künstliches Licht, welches hohe UV-Anteile aufweist, allein oder zusammen mit für kosmetische oder pharmazeutische Zubereitungen bekannten, im UV-Bereich absorbierenden Verbindungen. Unter organischen Materialien sind im weitesten Sinne somit auch die menschliche Haut und menschliche Haare zu verstehen. Die kosmetischen und pharmazeutischen Zubereitungen als solche werden zugleich natürlich auch stabilisiert, um möglichst lange wirksam zu bleiben.

Demgemäß sind auch Gegenstand der vorliegenden Erfindung kosmetische und pharmazeutische Zubereitungen zum Schutz der menschlichen Haut oder menschlicher Haare gegen UV-Licht im Bereich von 280 bis 400 nm, welche 0,01 bis 10 Gew.-%, vorzugswiese 0,1 bis 8 Gew.-%, besonders bevorzugt 2 bis 7 Gew.-%, bezogen auf die Menge der kosmetischen oder pharmazeutischen Zubereitung, eines oder mehrerer Isoindolinonderivate I allein oder zusammen mit für kosmetische oder pharmazeutische Zubereitungen bekannten, im UV-Bereich absorbierenden Verbindungen als Lichtschutzmittel enthalten.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-Bereich absorbierenden Substanzen mitverwendet werden, sofern sie im Gesamtsystem der erfindungsgemäß zu verwendenden Zubereitung photostabil sind.

Als UV-Filtersubstanzen, die in Kombination mit den erfindungsgemäß zu verwendenden Verbindungen der Formel I angewandt werden, kommen beliebige UV-A- und UV-B-Filtersubstanzen in Betracht. Beispielsweise sind zu nennen:

**Tabelle 1**

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4 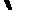 -methoxy-dibenzoylmethan | 70356-09-1 |
| 29 | 2,2 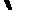 ,4,4 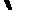 -Tetrahydroxybenzophenon | 131-55-5 |

Zum Schutz menschlicher Haare vor UV-Strahlen können die erfindugsgemäßen Lichtschutzmittel der Formel I in Shampoos, Lotionen, Gelen, Haarsprays, Aerosol-Schaumcremes oder Emulsionen in Konzentrationen von 0,01 bis 10 Gew.-%, bevorzugt 0,1 bis 8 Gew.-%, besonders bevorzugt 2 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilitat aus, und die mit I hergestellten Zubereitungen durch ihr angenehmes Hautgefühl aus.

Gegenstand der Erfindung sind auch die Verbindungen der Formel I zur Verwendung als Medikament sowie pharmazeutische Zubereitungen zur vorbeugenden Behandlung von Entzündungen und Allergien der Haut sowie zur Verhütung bestimmter Hautkrebsarten, welche eine wirksame Menge mindestens einer Verbindung der Formel I als Wirkstoff enthalten.

Das erfindungsgemäße pharmazeutische Mittel kann oral oder topisch verabreicht werden. Für die orale Verabreichung liegt das pharmazeutische Mittel in Form von u.a. Pastillen, Gelatinekapseln, Dragees, als Sirup, Lösung, Emulsion oder Suspension vor. Die topische Anwendung der pharmazeutischen Mittel erfolgt beispielsweise als Salbe, Creme, Gel, Spray, Lösung oder Lotion.

Die erfindungsgemäß zu verwendenden Verbindungen der Formel I können gemäß Chem. Ber. 1967, 2261 - 2273 entsprechend der folgenden Reaktionsgleichung 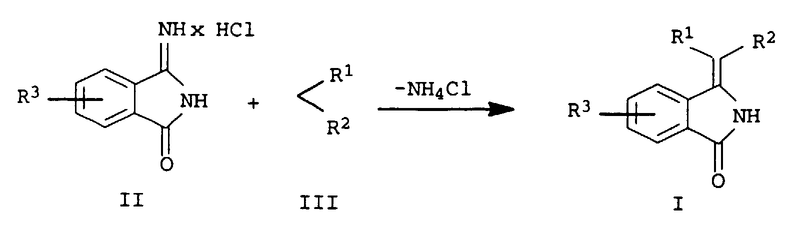 durch Kondensation von Iminoisoindolinonhydrochlorid der Formel II mit CH-aciden Verbindungen der allgemeinen Formel III hergestellt werden, wobei die Substituenten R¹ bis R³ die im Anspruch 1 genannte Bedeutung haben.

Beispielsweise ergibt die Umsetzung von Iminoisoindolinonhydrochlorid mit Cyanessigsäure-2-ethylhexylester die Verbindung 1 in Tab. 2.

In den nachfolgenden Beispielen wird die Herstellung und Verwendung der erfindungsgemäßen Isoindolinonderivate näher erläutert.

### I. Herstellung

### Beispiel 1

### Herstellvorschrift für die Verbindung der Nr. 1 der Tabelle 2

0,1 mol Iminoisoindolinonhydrochlorid und 0,1 mol Cyanessigsäure-2-ethylhexylester wurden in 50 ml Isobutanol gelöst und 5 h auf Rückfluß erhitzt. Nach Entfernung des Lösungsmittels im Vakuum, wurde der Rückstand in Wasser aufgenommen und auskristallisiert. Man erhielt 20 g (60 % Ausbeute) der Verbindung 1 aus Tabelle 2.

Analog Beispiel 1 wurden die in Tabelle 2 aufgeführten Verbindungen 2 bis 5 hergestellt.

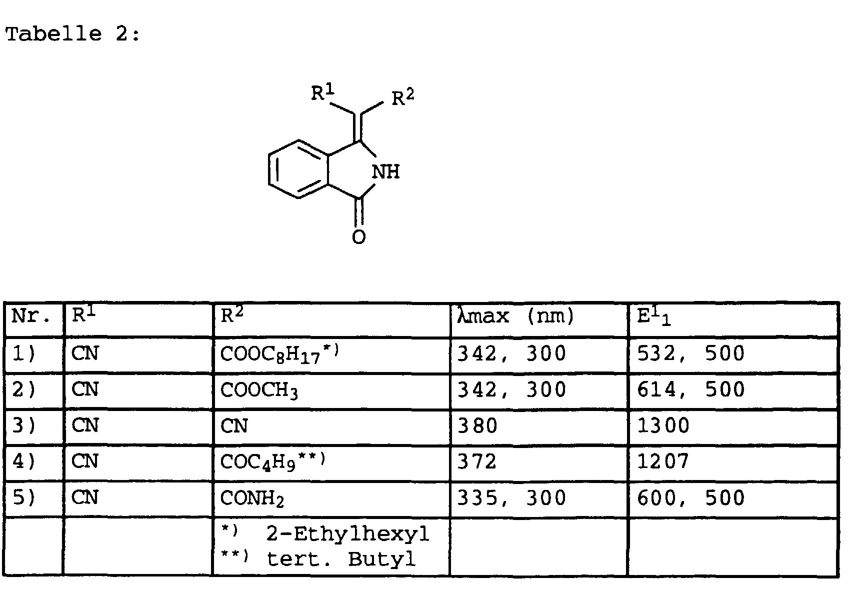

### II. Zubereitungen

### Allgemeine Vorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Beispiel 2

### Zusammensetzung für die Lippenpflege

| Massengehalt | |
|---|---|
| Gew.-% | |
| ad 100 | Eucerinum anhydricum |
| 10,00 | Glycerin |
| 10,00 | Titanium Dioxid |
| 5,00 | Verbindung Nr. 1 der Tabelle 2 |
| 8,00 | Octyl Methoxycinnamat |
| 5,00 | Zink Oxid |
| 4,00 | Castoröl |
| 4,00 | Pentaerythrithyl Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 3

### Zusammensetzung für die Lippenpflege

| Massengehalt | |
|---|---|
| Gew.-% | |
| ad 100 | Eucerinum anhydricum |
| 10,00 | Glycerin |
| 10,00 | Titanium Dioxid |
| 5,00 | Verbindung Nr. 2 der Tabelle 2 |
| 8,00 | Octyl Methoxycinnamat |
| 5,00 | Zink Oxid |
| 4,00 | Castoröl |
| 4,00 | Pentaerythrithyl Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 4

### Zusammensetzung für Sunblocker mit Mikropigmenten

| Massengehalt | |
|---|---|
| Gew.-% | |
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung Nr. 1 der Tabelle 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 5

### Zusammensetzung für Sunblocker mit Mikropigmenten

| Massengehalt | |
|---|---|
| Gew.-% | |
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung Nr. 2 der Tabelle 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 6

### Fettfreies Gel

| Massengehalt | |
|---|---|
| Gew.-% | |
| ad 100 | Wasser |
| 8,00 | Octyl Methoxycinnamat |
| 7,00 | Titanium Dioxid |
| 5,00 | Verbindung Nr. 1 der Tabelle 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 7

### Fettfreies Gel

| Massengehalt | |
|---|---|
| Gew.-% | |
| ad 100 | Wasser |
| 8,00 | Octyl Methoxycinnamat |
| 7,00 | Titanium Dioxid |
| 5,00 | Verbindung Nr. 2 der Tabelle 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 8

### Sonnencreme (LSF 20)

| Massengehalt | |
|---|---|
| Gew.-% | |
| ad 100 | Wasser |
| 8,00 | Octyl Methoxycinnamat |
| 8,00 | Titanium Dioxid |
| 6,00 | PEG-7-Hydrogenated Castor Öl |
| 5,00 | Verbindung Nr. 1 der Tabelle 2 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 9

### Sonnencreme (LSF 20)

| Massengehalt | |
|---|---|
| Gew.-% | |
| ad 100 | Wasser |
| 8,00 | Octyl Methoxycinnamat |
| 8,00 | Titanium Dioxid |
| 6,00 | PEG-7-Hydrogenated Castor Öl |
| 5,00 | Verbindung Nr. 2 der Tabelle 2 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 10

### Sonnencreme wasserfest

| Massengehalt | |
|---|---|
| Gew.-% | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 1 der Tabelle 2 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 11

### Sonnencreme wasserfest

| Massengehalt | |
|---|---|
| Gew.-% | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 2 der Tabelle 2 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 12

### Sonnenmilch (LSF 6)

| Massengehalt | |
|---|---|
| Gew.-% | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 1 der Tabelle 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

### Beispiel 13

### Sonnenmilch (LSF 6)

| Massengehalt | |
|---|---|
| Gew.-% | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 2 der Tabelle 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

### Beispiel 14

### Stabilisierung von Polystyrol

Zur Beurteilung der Stabilisatorwirkung der UV-Absorber wurden diese in einer Konzentration von 0,2 Gew.-% in Polystyrol 168 N (Fa. BASF) eingearbeitet. Dazu wurden das Polystyrol und der Stabilisator vorgemischt und über einen Einschneckenextruder bei einer Massetemperatur von 200°C gelöst und granuliert. Von dem anfallenden Granulat wurden mit einer Spritzgußmaschine bei 200°C Prüfkörper hergestellt (60x45x2 mm). Diese wurden in einem Schnellbewitterungsgerät Xenotest® 1200 (Fa. Hanau) bis 1000 Stunden künstlich bewittert. An den Proben wurde der Yellowness Index (YI) gemäß Annual Book of ASTM Standards D 1925-70 (Reapproved 1977) als Maß für den Vergilbungsgrad bestimmt. Die Ergebnisse sind in Tabelle 3 zusammengestellt.

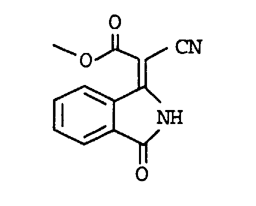

**Tabelle 3**

| YI-Werte von Polystyrol | | |
|---|---|---|
| | YI-Werte Belichtungszeit | |
| | 500 h | 1000 h |
| stabilisiert mit Verbindung 2 aus Tabelle 2 | 10 | 18 |
| Vergleichsversuch ohne Stabilisator | > 50 | |

## Patentansprüche

1. Verwendung von Isoindolinonderivaten der allgemeinen Formel I, 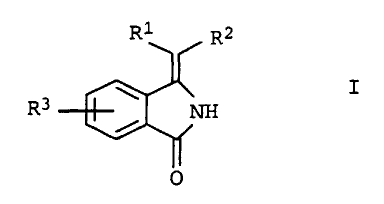 in der die Variablen unabhängig voneinander folgende Bedeutung haben:
R¹ Wasserstoff, COOR⁴, COR⁴, CONR⁴R⁵, CN, C₁-C₂₀-Alkyl, C₂-C₁₀-Alkenyl, C₃-C₁₀-Cycloalkyl, C₃-C₁₀-Cycloalkenyl, Aryl, Heteroaryl, gegebenenfalls substituiert;
R² COOR⁴, COR⁴, CONR⁴R⁵, CN;
R¹ und R² gemeinsam C₇-C₁₀-Bicycloalkyl, C₇-C₁₀-Bicycloalkenyl;
R³ Wasserstoff, C₁-C₂₀-Alkyl, C₂-C₁₀-Alkenyl, C₃-C₁₀-Cycloalkyl, C₃-C₁₀-Cycloalkenyl, C₁-C₁₂-Alkoxy, C₁-C₂₀-Alkoxycarbonyl, C₁-C₁₂-Alkylamino, C₁-C₁₂-Dialkylamino, Aryl, Heteroaryl, gegebenenfalls substituiert, wasserlöslich machende Substituenten, ausgewählt aus der Gruppe bestehend aus Carboxylat-, Sulfonat- oder Alkylammoniumresten;
R⁴ und R⁵ unabhängig voneinander Wasserstoff, C₁-C₂₀-Alkyl, C₂-C₁₀-Alkenyl, C₃-C₁₀-Cycloalkyl, C₃-C₁₀-Cycloalkenyl; Aryl, Heteroaryl, gegebenenfalls substituiert;
als Stabilisatoren für organische Materialien.

2. Verwendung von Isoindolinonderivaten der Formel I nach Anspruch 1, in der die Variablen unabhängig voneinander folgende Bedeutung haben:
R¹ und R² COOR⁴, COR⁴, CONR⁴R⁵, CN;
R³ Wasserstoff, C₁-C₈-Alkyl, C₁-C₈-Alkoxy, C₁-C₂₀-Alkoxycarbonyl, C₁-C₁₂-Alkylamino, C₁-C₁₂-Dialkylamino, Aryl, Heteroaryl, gegebenenfalls substituiert, wasserlöslich machende Substituenten, ausgewählt aus der Gruppe bestehend aus Carboxylat-, Sulfonat- oder Alkylammoniumresten;
R⁴ und R⁵ Wasserstoff, C₁-C₈-Alkyl, C₅-C₆-Cycloalkyl, Phenyl;

3. Verwendung von Isoindolinonderivaten der Formel I nach Anspruch 1, in der die Variablen unabhängig voneinander folgende Bedeutung haben:
R¹ und R² COOR⁴, COR⁴, CONR⁴R⁵, CN;
R³ Wasserstoff, C₁-C₈-Alkyl, C₁-C₈-Alkoxy, C₁-C₂₀-Alkoxycarbonyl, C₁-C₁₂-Alkylamino, C₁-C₁₂-Dialkylamino, Aryl, Heteroaryl, gegebenenfalls substituiert, Sulfonatrest;
R⁴ und R⁵ Wasserstoff, C₁-C₈-Alkyl;

4. Verwendung von Isoindolinonderivaten der Formel I nach den Ansprüchen 1 bis 3 als Stabilisatoren für Kunststoffe und Lacke.

5. Verwendung von Isoindolinonderivaten der Formel I nach den Ansprüchen 1 bis 3 als Stabilisatoren für kosmetische und pharmazeutische Zubereitungen.

6. Verwendung von Isoindolinonderivaten der Formel I nach den Ansprüchen 1 bis 5 als photostabile UV-Filter.

7. Verwendung von Isoindolinonderivaten der Formel I gemäß den Ansprüchen 1 bis 3 als photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

8. Organische Materialien, enthaltend 0,01 bis 10 Gew.-%, bezogen auf die Menge des organischen Materials, eines oder mehrerer Isoindolinonderivate der Formel I gemäß Anspruch 1.

9. Kunststoffe und Lacke, enthaltend ein oder mehrere Isoindolinonderivate der Formel I gemäß Anspruch 1.

10. Kunststoffe und Lacke nach Anspruch 9, enthaltend 0,01 bis 10 Gew.-%, bezogen auf die Menge des Kunststoffes bzw. Lackes, eines oder mehrerer Isoindolinonderivate der Formel I gemäß Anspruch 1.

11. Lichtschutzmittel enthaltende kosmetische und pharmazeutische Zubereitungen, dadurch gekennzeichnet, daß sie in einem kosmetisch und pharmazeutisch geeigneten Träger, allein oder zusammen mit für kosmetische und pharmazeutische Zubereitungen bekannten im UV-Bereich absorbierenden Verbindungen, als photostabile UV-Filter wirksame Mengen von Isoindolinonderivaten der Formel I gemäß Anspruch 1 enthalten.

12. Isoindolinonderivate der Formel I gemäß Anspruch 1 zur Verwendung als Arzneimittel.

13. Pharmazeutische Zubereitungen, enthaltend eine wirksame Menge mindestens einer der Isoindolinonderivate der Formel I gemäß Anspruch 1.
